# EUROPÄISCHE PATENTSCHRIFT

(11) **EP 2 329 776 B1**
(45) Veröffentlichungstag und Bekanntmachung des Hinweises auf die Patenterteilung: **26.09.2012**
(21) Anmeldenummer: 10015059.8
(22) Anmeldetag: 27.11.2010
(51) Int. Cl.: A61B 17/42, A61B 19/00, A61B 17/00

(54) **Uterusmanipulator**
Uterus manipulator
Manipulateur pour utérus

(30) Priorität: 02.12.2009 DE 102009056705
(43) Veröffentlichungstag der Anmeldung: 08.06.2011
(73) Patentinhaber: Richard Wolf GmbH, 75438 Knittlingen (DE)
(72) Erfinder: Boebel, Manfred, 75245 Bauschlott (DE); Weber, Bernd Claus, Dr., 76228 Karlsruhe (DE); Solima, Eugenio, Dr., 20122 Milano (IT)
(74) Vertreter: Vollmann, Heiko

(56) Entgegenhaltungen:
- EP-A2- 2 060 236
- DE-A1- 19 543 576
- DE-U1- 8 529 364
- US-A- 5 840 077
- US-A1- 2010 106 163

## Beschreibung

Die Erfindung betrifft einen Uterusmanipulator, insbesondere für die laparoskopisch assistierte vaginale Hysterektomie mit den im Oberbegriff des Anspruchs 1 angegebenen Merkmalen.

Bei der kombiniert laparoskopisch und durch die Scheide durchgeführten operativen Entfernung der Gebärmutter (LAVH-laparoskopisch assistierte vaginale Hysterektomie) ist ein Instrumentensatz erforderlich, der unter anderem einen Uterusmanipulator umfasst, mit welchem der Uterus von Außen durch die Scheide mittels einer durch den Cervikalkanal eingeführten Sonde geführt wird. Ein solcher Uterusmanipulator, von dem die Erfindung ausgeht, ist beispielsweise aus US 5,520,698 A Basis für den Oberbegriff, des Anspruchs 1 bekannt. Dieses Instrument weist zusätzlich zur Sonde noch eine distalseitig offene Glocke auf, welche zur Aufnahme der Cervix dient und die Führung der Gebärmutter durch das Instrument verbessert. Die Glocke ist mit seitlichen Durchbrechungen versehen. Mit Abstand zur Glocke ist proximalseitig dazu eine Vaginaldichtung vorgesehen, welche durch einen aufblasbaren Ballonring gebildet ist, der sich dichtend an die Scheidenwand anlegen soll und dafür sorgt, dass nach dem Durchtrennen des hinteren Scheidengewölbes das Pneumoperitoneum aufrecht erhalten wird, um damit einem Kollabieren des Abdomens entgegenzuwirken.

Ein weiterer Uterusmanipulator ist aus DE8529364 U1 bekannt. Dieser Uterusmanipulator offenbart eine distalwärts offenen Glocke zur Aufnahme einer Cervix, mit einem Saugkanal und mit mindestens einer Saugöffnung im distalen Endabschnitt, wobei die Glocke als Saugglocke ausgebildet ist und mindestens ein Saugkanal in der Glocke mündet.

Derartige Uterusmanipulatoren zählen zum Stand der Technik und werden von unterschiedlichen Herstellern in unterschiedlichen Ausführungen angeboten. Dabei zählt es weiterhin zum Stand der Technik, die Sonde als Hohlsonde auszubilden und damit während des Eingriffs zur Vermeidung von Streuung karzinogener Zellen eine Absaugung intrauteriner Zellen durchzuführen.

Es hat sich jedoch gezeigt, dass diese intrauterine Zellabsaugung insbesondere bei dem vorbeschriebenen Instrument, bei dem zur Fixierung der Sonde innerhalb des Uterus dort ebenfalls ein Ballon aufgeblasen wird, unzureichend ist. Das Aufblasen dieses inneren Ballons ist hingegen erforderlich, um einen sicheren Halt der Sonde innerhalb des Uterus zu gewährleisten und damit eine sichere Führung des Uterus von außen mittels des Manipulators zu gewährleisten.

Vor diesem Hintergrund liegt der Erfindung die Aufgabe zugrunde, einen gattungsgemäßen Uterusmanipulator hinsichtlich seiner Funktionalität und Handhabung zu verbessern sowie insbesondere die vorgenannten Nachteile zu vermeiden.

Diese Aufgabe wird gemäß der Erfindung durch die in Anspruch 1 angegebenen Merkmale gelöst. Vorteilhafte Ausgestaltungen der Erfindung sind in den Unteransprüchen, der nachfolgenden Beschreibung sowie der Zeichnung angegeben.

Der erfindungsgemäße Uterusmanipulator, der insbesondere für die laparoskopisch assistierte vaginale Hysterektomie bestimmt ist, weist einen Schaft auf sowie einen distalen Endabschnitt, der zum Einführen in einen Uterus durch den Cervikalkanal bestimmt und ausgebildet ist. Er weist darüber hinaus eine proximale Handhabe auf sowie eine distalwärts offene Glocke zur Aufnahme einer Cervix. Proximalwärts versetzt zu dieser Glocke ist eine Vaginaldichtung angeordnet. Der Uterusmanipulator weist weiterhin mindestens einen Saugkanal auf, der mit mindestens einer Saugöffnung im distalen Endabschnitt leitungsverbunden ist. Gemäß der Erfindung ist dabei die Glocke als Saugglocke ausgebildet. Hierzu weist der Uterusmanipulator einen Saugkanal auf, der in der Glocke mündet. Dabei ist erfindungsgemäß die Vaginaldichtung durch eine nach proximalwärts offene Dichtglocke gebildet, deren umlaufender Rand zur dichtenden Anlage an der Vaginalwand ausgebildet ist. Erfindungsgemäß sind Saugglocke und Dichtglocke als Baueinheit oder zumindest Teil einer Baueinheit ausgebildet, die lösbar am Schaft befestigt ist.

Grundgedanke der erfindungsgemäßen Lösung ist es, durch die Saugglocke, also eine über einen Saugkanal und eine daran anschließende Saugleitung eine mit Unterdruck beaufschlagte Saugglocke vorzusehen, so dass eine Absaugung etwaiger karzinogener Zellen nicht nur aus dem Uterus heraus über die Saugöffnung im distalen Endabschnitt sondern darüber hinaus auch extern im Bereich der Portio mittels der Saugglocke erfolgen kann. Dabei dient die erfindungsgemäße Saugglocke nicht nur der Zellabsaugung im Bereich der Portio sondern darüber hinaus auch der Fixierung am Uterusmanipulator, so dass eine mechanische Verankerung des distalen Endabschnitts innerhalb des Uterus entbehrlich ist. Es können somit mit dem erfindungsgemäßen Uterusmanipulator von außen Zugkräfte aufgebracht werden, ohne dass die Gefahr besteht, dass der distale Endabschnitt aus dem Cervikalkanal herausgleitet. Darüber hinaus ist sichergestellt, dass selbst die Zellen, die zwischen dem distalen Endabschnitt des Instrumentes und den Cervikalkanal gelangen durch den an die Saugglocke anschließenden Saugkanal abgesaugt werden und somit nicht in den Bereich der Scheide gelangen.

Dadurch, dass die Vaginaldichtung durch eine nach proximalwärts offene Dichtglocke gebildet ist, deren umlaufender Rand zur dichtenden Anlage an der Vaginalwand ausgebildet ist, ist eine gemeinsame Anbringung der Bauteile am Instrument möglich, gleichzeitig jedoch der erforderliche Abstand gebildet, der erforderlich ist, damit nach Durchtrennung des hinteren Scheidengewölbes die Dichtwirkung erhalten bleibt.

Dadurch, dass Saugglocke und Dichtglocke, welche die Vaginaldichtung bildet, eine Baueinheit oder zumindest Teil einer Baueinheit bilden, die lösbar am Schaft befestigt ist, kann diese als Einwegartikel ausgebildet sein und in steriler Verpackung bevorratet werden, also erst unmittelbar vor Gebrauch mit dem Instrument verbunden werden. Dabei ist es besonders vorteilhaft, wenn nur eine gemeinsame Verbindung dieser Baueinheit mit dem Schaft vorgesehen ist, da dies die Handhabung vereinfacht.

Typischerweise handelt es sich bei dem distalen Endabschnitt um den Endabschnitt einer Hohlsonde. Dabei kann in einfachster Form die Hohlsonde durch den Schaft selbst gebildet sein. Typischerweise und bevorzugt ist jedoch die Hohlsonde innerhalb des Instrumentschaftes beweglich geführt.

Gemäß einer vorteilhaften Weiterbildung der Erfindung weist die Saugglocke an ihrer Innenseite, also am Innenumfang mindestens eine, vorzugsweise mehrere Abstufungen auf. Die Abstufung dient zur Anpassung des Instruments an unterschiedliche Portiogrößen, so dass sichergestellt ist, dass die Portio stets voll umfänglich an der Saugglocke dicht und mechanisch fest anliegt.

Besonders vorteilhaft ist es, wenn die Saugglocke einen distalen umlaufenden Rand aufweist und Leuchtmittel zur Beleuchtung dieses Randes vorgesehen sind. Dabei ist die Glocke zweckmäßigerweise aus einem durchsichtigen Material, so dass sie unter Sichtkontrolle angebracht werden kann. Die Beleuchtung des umlaufenden Randes kann gemäß der Erfindung entweder durch im Bereich der Glocke angebrachte Leuchtmittel, typischerweise Leuchtdioden, oder aber mittels Lichtleitern erfolgen, die an die Saugglocke anschließen und vom proximalen Instrumentenende her über eine Lichtquelle lichtversorgt werden.

Die Beleuchtung dieses umlaufenden Randes kann zwar zum Handhaben des Manipulators, insbesondere zum Einführen des distalen Endabschnittes in den Cervikalkanal vorteilhaft sein, der wesentliche Vorteil liegt jedoch darin, dass das hintere Scheidengewölbe dort, wo es durch einen umlaufenden Schnitt getrennt werden soll, beleuchtet ist, so dass der Operateur zum einem eine bessere Orientierung hat, zum anderen auch Gefäße oder ähnliches sichtbar werden, die dort liegen. Die Saugglocke sollte dazu zweckmäßigerweise aus lichtleitendem Material bestehen, bevorzugt aus klarem durchsichtigen Glas oder Kunststoff.

Dabei hat es sich als besonders vorteilhaft erwiesen, wenn die Saugglocke am Außenumfang kegelstumpfförmig und am Innenumfang mehrfach abgestuft ausgebildet ist und die Lichtbeaufschlagung an einer Stirnfläche nahe dem schaftseitigen Innenumfang erfolgt. Dabei dient die Mehrfachabstufung der Innenseite der Saugglocke zur Anpassung an anatomische Größenunterschiede, wohingegen die Außenform im Wesentlichen zur Übertragung des Lichts durch Totalreflektion an den Wänden von einer proximalen Stirnfläche nahe dem schaftseitigen Innenumfang zum distalseitigen Ring hin dient. Die Ausnutzung der Totalreflektion ist besonders vorteilhaft, jedoch nicht notwendig, es kann auch in diesem Bereich eine Lichtleitung über Lichtleiter oder andere geeignete Ausbildungen der Glockenwandung erfolgen.

Hinsichtlich der Funktionalität ist es besonders vorteilhaft, wenn die Saugglocke durch ein möglichst eigensteifes stabiles Bauteil gebildet wird, wohingegen die Vaginaldichtung zumindest im Umfangsbereich möglichst weichelastisch ausgebildet sein sollte. Dabei kann die Saugglocke vorteilhaft als Kunststoffspritzgussteil ausgebildet sein. Besonders zweckmäßig ist es, wenn nicht nur die Saugglocke sondern auch der Teil der Baueinheit, welcher zur Befestigung dient (der natürlich auch Teil der Glocke sein kann) sowie gegebenenfalls auch noch der innere Teil der Dichtglocke als Kunststoffspritzgussteil ausgebildet ist und der äußere Teil der Dichtglocke, zumindest die Vaginaldichtung aus einem weichelastischen z. B. Silikonteil hergestellt ist. Die Bauteile können stoffschlüssig zu einer Baueinheit verbunden sein, es ist jedoch auch eine formschlüssige Verbindung nach einer Wulst-Nut-Verbindung denkbar, wie dies bei Dichtungen an sich bekannt ist oder eine Kombination davon.

Vorteilhaft ist gemäß einer Weiterbildung der Erfindung in dem Schaft des Instrumentes drehfest jedoch axial verschiebbar eine Hohlsonde geführt, welche den distalen Endabschnitt zum Einführen in den Cervikalkanal aufweist und die in mindestens zwei unterschiedlichen Axialstellungen im Bezug auf den Schaft und/oder die proximale Handhabe fixierbar ist. Der über den Schaft hinausragende Teil der Hohlsonde bildet den distalen Endabschnitt, welcher zum Einführen in den Cervikalkanal vorgesehen ist. Um hier das Instrument an die anatomischen Größenverhältnisse anzupassen, ist die Hohlsonde axial verschiebbar und in unterschiedlichen Stellungen fixierbar angeordnet, um damit die Länge des distalen Endabschnittes individuell anpassen zu können. Dabei ist die Hohlsonde drehfest innerhalb des Instrumentes angeordnet, um die beim Eingriff erforderlichen Manipulationen nicht nur in Achsrichtung sondern auch in Drehrichtung um die Achse herum vornehmen zu können.

Zweckmäßigerweise ist die Saugglocke, vorzugsweise die gesamte Baueinheit, mit Saugglocke und Vaginaldichtung nahe dem distalen Schaftende auf diesem befestigt, beispielsweise durch eine Schraubverbindung oder eine Bajonettverbindung. Dies vereinfacht den Aufbau des Instrumentes insbesondere auch die Montage und Demontage, wie sie regelmäßig nach bzw. vor einem Eingriff erforderlich ist, um das Instrument zu reinigen und zu sterilisieren.

Als Handhabe wird vorteilhaft ein Griffteil eingesetzt, welches fest mit dem Schaft verbunden ist, so dass der Schaft proximalseitig dieses Griffteil trägt.

Um einerseits das den Schaft überragende Ende der Hohlsonde, also den in den Cervikalkanal einführbaren distalen Endabschnitt in seiner Länge variieren zu können, um an die anatomischen Erfordernisse anzupassen, andererseits jedoch diesen zuverlässig festlegen zu können, so dass er drehfest und auch in Achsrichtung fest mit dem Griffteil bzw. dem Schaft verbunden ist, ist gemäß einer Weiterbildung vorgesehen, die Hohlsonde am Außenumfang mit Rastausnehmungen zu versehen und innerhalb des Griffteils einen federvorgespannten Rastköper vorzusehen, derart, dass dieser Rastkörper in eine der Ausnehmungen eingreift und damit je nach Wahl der Ausnehmung die axiale Erstreckung der Hohlsonde vom Griffteil bzw. vom distalen Schaftende festlegt und im übrigen formschlüssig festlegt. Um dabei möglichst gut von außen die Länge des den Schaft überragenden distalen Endabschnittes der Hohlsonde erfassen zu können, ist gemäß einer Weiterbildung der Erfindung die Hohlsonde so ausgebildet, dass sie den Griffteil proximal überragt und dort mit einer Skalierung versehen ist, welche die freie Länge des distalen Endabschnitts entsprechend der axialen Position der Hohlsonde anzeigt. Es kann also bei dieser Ausbildung proximalseitig am Instrument abgelesen werden, welche Länge der distale Endabschnitt aufweist. Da die Rastmechanik innerhalb des Griffteils vorgesehen ist, kann diese Länge des distalen Endabschnitts gegebenenfalls auch noch während des Eingriffs korrigiert oder angepasst werden.

Gemäß einer vorteilhaften Weiterbildung der Erfindung weist die Hohlsonde einen zentralen Saugkanal auf, welcher im Endbereich des

Schaftes über eine Querdurchbrechung in der Sondenwandung in einem zwischen der Hohlsonde und dem Schaft gebildeten Ringkanal mündet, der mittels eines O-Rings abgedichtet ist und distalseitig in der Saugglocke mündet. Der Ringkanal ist über die damit leitungsverbundene Hohlsonde an eine Saugleitung angeschlossen. Eine solche Saugleitung kann an einem entsprechenden Anschlussstutzen am proximalen Ende der Hohlsonde anschließen, so dass innerhalb des Instrumentes praktisch keine Leitungen zu führen sind, was von Vorteil ist.

Zur Beleuchtung der Saugglocke, insbesondere des distalseitigen umlaufenden Ringes, sind entweder im oder am Schaft Lichtleiter angeordnet, die an einer Stirnfläche nahe dem Innenumfang der Saugglocke münden und die proximalseitig über ein Lichtleitkabel mit einer stationären Lichtquelle verbunden sind oder aber, was gemäß einer Weiterbildung der Erfindung alternativ vorgesehen ist, sind in oder an der Saugglocke Leuchtdioden oder andere geeignete Beleuchtungsmittel vorgesehen, die über im oder am Schaft geführte elektrische Versorgungsleitungen mit elektrischer Energie versorgt werden.

Die Erfindung ist nachfolgend anhand eines in der Zeichnung dargestellten Ausführungsbeispiels näher erläutert. Es zeigen:
- Fig. 1: in stark vereinfachter schematischer Darstellung einen Uterusmanipulator im Längsschnitt,

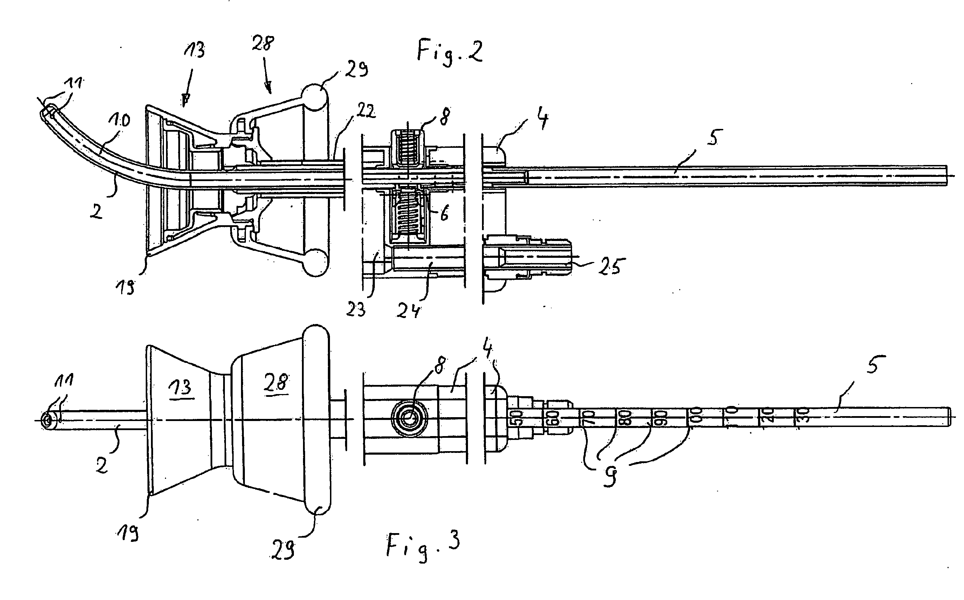

- Fig. 2: in vergrößerter schematisierter Schnittdarstellung den distalen, proximalen und einen mittleren Teil des Instrumentes,
- Fig. 3: eine Ansicht der Instrumententeile gemäß Figur 2,

- Fig. 4: in stark vergrößerter schematischer Darstellung einen mittleren Abschnitt des Instrumentes im Bereich des Griffteils im Schnitt,
- Fig. 5: den distalen Instrumententeil in einer ersten Ausführungsvariante im Längsschnitt,
- Fig. 6: einen Schnitt durch den Schaft im distalen Endbereich,
- Fig. 7: eine andere Ausführungsform in Darstellung nach Figur 5 und
- Fig. 8: die entsprechende Schnittdarstellung entsprechend Figur 6.

Der anhand der Figuren dargestellte Uterusmanipulator weist eine Hohlsonde 1 auf, die sich durch das gesamte Instrument erstreckt und am distalen Ende einen distalen Endabschnitt 2 bildet, der zum Einführen in einen Uterus durch den Cervikalkanal bestimmt ist. Die Hohlsonde 1 erstreckt sich vom distalen Endabschnitt 2 proximalwärts durch einen Schaft 3, der proximalseitig in einem Griffteil 4 festgelegt ist, welches die Handhabe des Instrumentes bildet. Die Hohlsonde 1 erstreckt sich durch den Griffteil 4 hindurch und weist einen proximalen Endabschnitt 5 auf, welcher das Griffteil 4 proximalseitig überragt und dort hohl ausgebildet ist. Die Hohlsonde 1 ist drehfest jedoch in Achsrichtung lösbar mit dem Griffteil 4 verbunden.

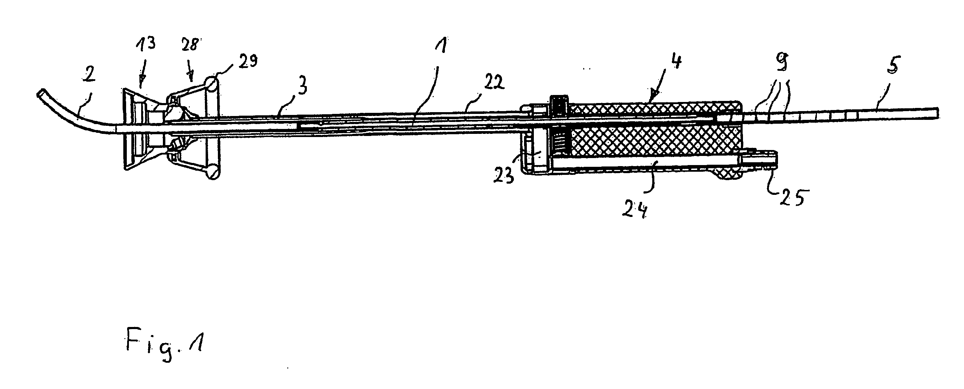

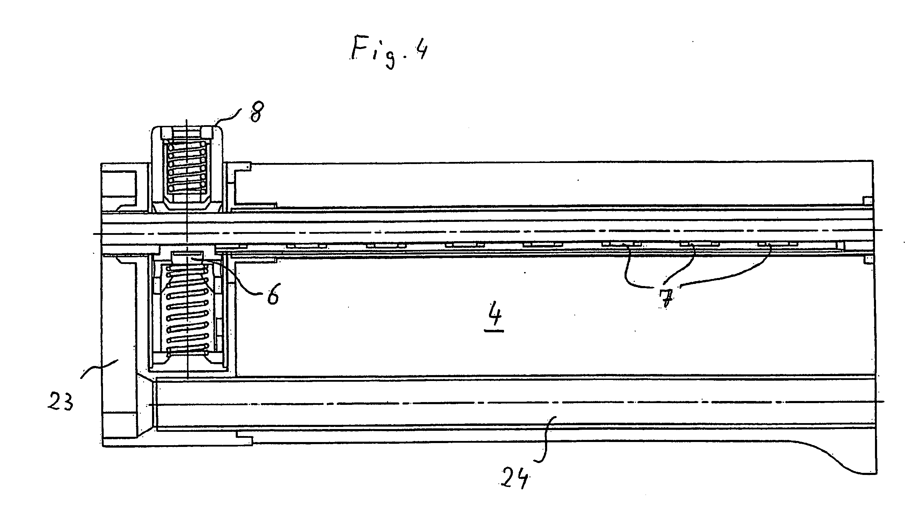

Die Länge des distalen Endabschnittes 2, also die Länge mit der die Hohlsonde 1 den Schaft 3 distal überragt, ist einstellbar, um das Instrument insbesondere die vorgenannte Länge an die anatomischen Verhältnisse anpassen zu können. Hierzu ist innerhalb des Griffteils 4 ein federvorgespannter Rastkörper 6 vorgesehen, der wahlweise in eine von einer Vielzahl über die Länge der Hohlsonde 1 im Bereich des Griffteils 4 angeordneter Rastausnehmungen 7 eingreifen kann. Durch Eindrücken einer am Griffteil 4 vorgesehenen Taste 8 gegen Federkraft wird der Rastkörper 6 in Figur 1 und 4 nach unten aus der Rastaufnehmung 7 herausbewegt, wonach die Hohlsonde 1 in axialer Richtung verschiebbar ist. Nach Loslassen der Taste 8 rastet der Rastkörper 6 in der nächst vorbeilaufenden Rastausnehmung 7 ein und zwar derart, dass die Hohlsonde 1 formschlüssig, nicht nur in Drehrichtung sondern auch in Achsrichtung im Griffteil 4 fixiert ist. Dabei sind an dem proximalen Endabschnitt 5 entsprechend dem axialen Abstand der Rastausnehmungen 7 Markierungen 9 vorgesehen, die beispielsweise mit fortlaufenden Zahlen versehen sind, so dass anhand dieser Markierungen der Operateur ermessen kann, welche Länge der damit eingestellte distale Endabschnitt 2 aufweist.

Die Hohlsonde 1 weist über ihre gesamte Länge, also auch im Bereich des Schaftes 3 bis zum distalen Endabschnitt 2, einen zentralen Saugkanal 10 auf, der in seitlichen und einer stirnseitigen Öffnung 11 am distalen Ende des Endabschnitts 2 mündet. Dieser Saugkanal 10 ist im Bereich des Schaftes 3, und zwar etwa in der Mitte zwischen dem distalen Schaftende und dem Griffteil 4, mit einer (nicht dargestellten) Ausnehmung in der Wandung versehen, welche diesen Saugkanal 10 mit einem zwischen der Außenseite der Hohlsonde 1 und dem Schaft 3 gebildeten Ringkanal 12 verbindet, der proximalseitig über einen O-Ring abgedichtet und mit einem (ebenfalls nicht dargestellten) Sauganschluss am proximalen Ende der Hohlsonde verbunden ist, über den eine Unterdruck führende Leitung anschließbar ist. Über die vorbeschriebene Leitungsverbindung erfolgt während des Eingriffs eine Absaugung aus dem Uterus, also der dort gelösten oder sich lösenden Zellen.

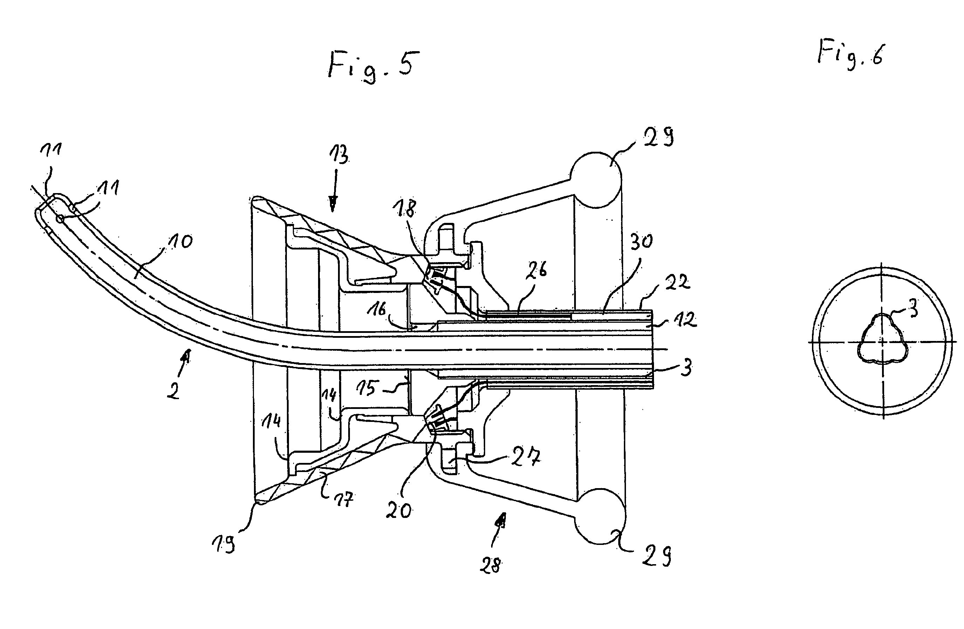

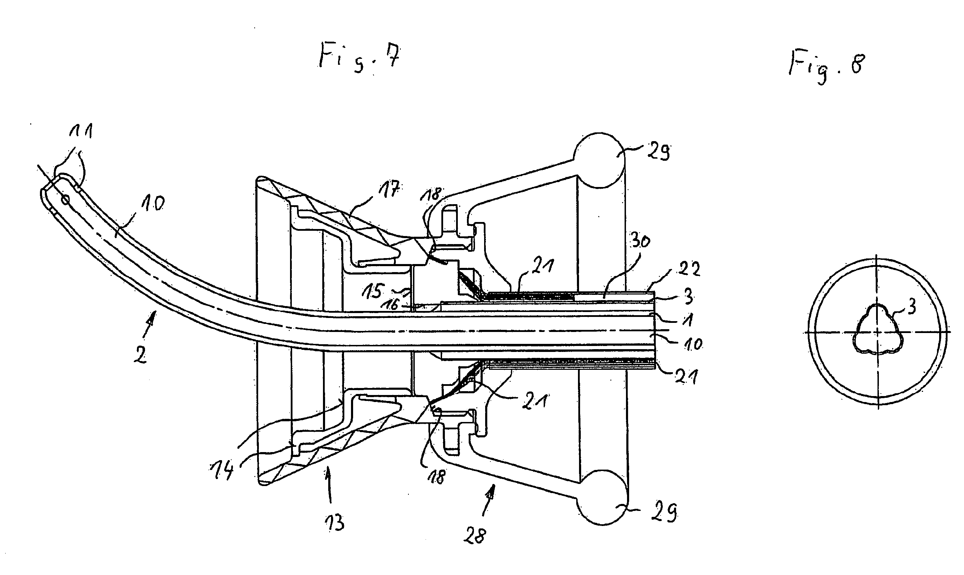

Um den Uterus sicher führen zu können, ist am distalen Ende des Schaftes 3 eine Saugglocke 13 vorgesehen, welche distalwärts geöffnet und zur Aufnahme der Portio vorgesehen und bestimmt ist. Die Saugglocke ist an ihrer Innenseite abgestuft ausgebildet, um den anatomischen Größenunterschieden gerecht zu werden. Bei den dargestellten Ausführungen sind zwei Stufen 14 vorgesehen. Dabei ragt die Saugglocke 13 bis an den Schaft 3 heran, auf dem sie schraubbefestigt ist. Im Boden 15 der Saugglocke 13 mündet der zwischen Hohlsonde 1 und Schaft 3 gebildete Ringkanal 12 in einer Durchbrechung 16, worüber die Innenseite der Saugglocke 13 mit dem Sauganschluss des Instrumentes verbunden ist. Hierdurch wird die Portio nicht nur dicht und fest in die Saugglocke 13 eingezogen sondern es wird darüber hinaus auch sichergestellt, dass sich dort lösende Zellen oder Zellen, die zwischen dem distalen Endabschnitt 2 und den Cervikalkanal gelangen, sicher abgesaugt werden. Die Hohlsonde 1 ist im Bereich des distalen Endes des Schaftes 3 durch 3 symmetrisch um die Längsachse verteilte Stifte 30 abgestützt, wie aus den Figuren 5 und 7 ersichtlich ist.

Während die Innenseite der Saugglocke 13 abgestuft ausgebildet ist, ist die Außenseite konisch zulaufend ausgebildet. Die Saugglocke 13 weist eine trichterförmig verlaufende Wandung 17 auf, die so gestaltet ist, dass an der proximalen Stirnseite 18 dieser Wandung 17 eingespeistes Licht durch Totalreflektion bis zu einem distalen Ring 19 geleitet wird, welcher beim Eingriff das hintere Scheidengewölbe beleuchtet und damit dem Operateur den Bereich markiert, der von der anderen Seite, also von abdominal her laparoskopisch aufzutrennen ist. Durch diesen distalen Ring 19 werden insbesondere in diesem Bereich liegende Gefäße sichtbar. Die Lichteinspeisung an der proximalen Stirnseite 18 erfolgt entweder mittels Leuchtdioden 20 (siehe Figur 5), die in dichter Anordnung in der Stirnseite der Wandung 17 eingegliedert sind oder aber über dort endende Lichtleiter 21, wie sie in Figur 7 dargestellt sind.

Die Lichtleiter 21 sind durch einen Ringkanal geführt, der zwischen dem Schaft 3 und einem diesem umgebenden Außenschaft 22 gebildet ist und der im Griffteil 4 endet. Dort sind die Lichtleiter über einen Querkanal 23 und einen daran um 90° anschließenden den Griffteil längs durchsetzenden Längskanal 24 geführt, an dessen Ende ein Lichtleiteranschluss 25 vorgesehen ist. Bei der Ausführung gemäß Figur 5 sind durch die vorbeschriebenen Kanäle statt der Lichtleiter elektrische Versorgungsleitungen 26 geführt, über welche die Leuchtdioden 20 elektrisch versorgt werden. Im Griffteil kann auch ein Akku oder ein Netzteil angeordnet sein.

Die Saugglocke 13, die am Ende des Schaftes 3 befestigt ist, ist aus durchsichtigem klaren Kunststoff gebildet, so dass der Operateur durch diese hindurchschauen und somit das Instrument sicher einführen und platzieren kann. Sie weist an ihrem Außenumfang, etwa in Höhe der Befestigung am Schaft 3, einen umlaufenden Wulst 27 auf, der in eine entsprechend ausgebildete Nut einer aus elastischem Material, typischerweise Silikon, gebildeten und proximalwärts offenen Dichtglocke 28 eingreift. Die Dichtglocke 28, die mit ihrer Nut den Wulst 27 formschlüssig umgreift, ist sowohl innen als auch außen proximalwärts aufweitend ausgebildet und endet in einem umlaufenden Dichtring 29, dem Vaginaldichtring. Dieser Dichtring 29 dichtet das Instrument gegenüber der Vaginalwand ab und sorgt dafür, dass beim Durchtrennen des hinteren Scheidengewölbes der im Körperinneren zwecks Laparoskopie aufgebaute Innendruck (Pneumoperitoneum) nicht über die Scheide entweicht. Auch die Dichtglocke 28 kann aus transparentem Kunststoff bestehen. Dichtglocke 28 und Saugglocke 13 bilden eine Baueinheit, die über die Saugglocke 13 am Schaft 3 befestigt ist und sich, wie die Figuren 5 und 7 verdeutlichen, saugglockenseitig auch am Außenschaft 22 abstützt. Diese Baueinheit ist als Einwegartikel konzipiert, wird also steril verpackt aufbewahrt, vor dem Eingriff am Instrument angebracht und nach dem Eingriff nach Demontage entsorgt. Dabei ist die Saugglocke 13 eigensteif ausgebildet, so dass sie sich praktisch wie ein starres Bauteil verhält, wohingegen die Dichtglocke 28 weichelastisch ist und sich dichtend an die Scheidenwand anlegt.

### Bezugszeichenliste

- 1 -: Hohlsonde
- 2 -: distaler Endabschnitt
- 3 -: Schaft
- 4 -: Griffteil
- 5 -: proximaler Endabschnitt
- 6 -: Rastkörper
- 7 -: Rastausnehmungen
- 8 -: Taste
- 9 -: Markierungen
- 10 -: Saugkanal
- 11 -: Öffnungen
- 12 -: Ringkanal
- 13 -: Saugglocke
- 14 -: Stufen
- 15 -: Boden
- 16 -: Durchbrechung
- 17 -: Wandung
- 18 -: proximale Stirnseite
- 19 -: distaler Ring
- 20 -: Leuchtdioden
- 21 -: Lichtleiter
- 22 -: Außenschaft
- 23 -: Querkanal
- 24 -: Längskanal
- 25 -: Lichtleiteranschluss
- 26 -: elektrische Leitung
- 27 -: Wulst
- 28 -: Dichtglocke
- 29 -: Vaginaldichtring
- 30 -: Stifte

## Patentansprüche

1. Uterusmanipulator , insbesondere für die laparoskopisch assistierte vaginale Hysterektomie, mit einem Schaft (3), mit einem distalen Endabschnitt (2), der zum Einführen in einen Uterus durch den Cervikalkanal bestimmt und ausgebildet ist, mit einer proximalen Handhabe (4), mit einer distalwärts offenen Glocke (13) zur Aufnahme einer Cervix und mit einer bezogen auf die Glocke (13) nach proximalwärts versetzt dazu angeordneten Vaginaldichtung (29), mit mindestens einem Saugkanal (10) und mit mindestens einer Saugöffnung (11) im distalen Endabschnitt (2), **dadurch gekennzeichnet, dass** die Glocke (13) als Saugglocke ausgebildet ist, und dass mindestens ein Saugkanal (12) in der Glocke (13) mündet, wobei die Vaginaldichtung (29) durch eine nach proximalwärts offene Dichtglocke (28) gebildet ist, deren umlaufender Rand (29) zur dichtenden Anlage an der Vaginalwand ausgebildet ist und wobei die Saugglocke (13) und die Dichtglocke (28) eine Baueinheit oder Teil einer Baueinheit bilden, die lösbar am Schaft (3) befestigt ist.

2. Uterusmanipulator nach Anspruch 1, **dadurch gekennzeichnet dass** die Saugglocke (13) an ihrem Innenumfang mindestens eine Abstufung (14) aufweist.

3. Uterusmanipulator nach einem der vorhergehenden Ansprüche, **dadurch gekennzeichnet dass** Leuchtmittel (20; 21) zur Beleuchtung des umlaufenden distalen Randes (19) der Saugglocke (13) vorgesehen sind.

4. Uterusmanipulator nach einem der vorhergehenden Ansprüche, **dadurch gekennzeichnet dass** die Saugglocke (13) aus lichtleitendem Material besteht, außen kegelstumpfförmig und innen mehrfach abgestuft ausgebildet ist, wobei die Lichtbeaufschlagung an einer Stirnfläche (18) nahe dem schaftseitigen Innenumfang erfolgt.

5. Uterusmanipulator nach einem der vorhergehenden Ansprüche, **dadurch gekennzeichnet dass** die Baueinheit ein zumindest die Saugglocke (13) bildendes eigensteifes Bauteil und ein zumindest die Vaginaldichtung (29) bildendes weichelastischen Bauteil aufweist.

6. Uterusmanipulator nach einem der vorhergehenden Ansprüche, **dadurch gekennzeichnet dass** das die Saugglocke (13) umfassende Bauteil als Kunststoffspritzgussteil ausgebildet ist.

7. Uterusmanipulator nach einem der vorhergehenden Ansprüche, **dadurch gekennzeichnet dass** in dem Schaft (3) drehfest und axial verschiebbar eine Hohlsonde (1) geführt ist, welche den distalen Endabschnitt (2) zum Einführen in den Cervikalkanal aufweist und die in mindestens zwei unterschiedlichen Axialstellungen in Bezug auf den Schaft (3) und/oder die proximale Handhabe (4) fixierbar ist.

8. Uterusmanipulator nach einem der vorhergehenden Ansprüche, **dadurch gekennzeichnet dass** die Saugglocke (13), insbesondere die Baueinheit nahe dem distalen Schaftende auf diesem befestigt ist.

9. Uterusmanipulator nach einem der vorhergehenden Ansprüche, **dadurch gekennzeichnet dass** der Schaft (3) proximalseitig ein Griffteil (4) trägt, welches die Handhabe des Instrumentes bildet.

10. Uterusmanipulator nach einem der vorhergehenden Ansprüche, **dadurch gekennzeichnet dass** die Hohlsonde (1) am Außenumfang Rastausnehmungen (7) aufweist und dass ein im Griffteil (4) angeordneter federvorgespannter Rastkörper (6) in eine der Ausnehmungen (7) eingreift und damit die axiale Erstreckung der Hohlsonde (1) vom Griffteil (4) und/oder dem distalen Schaftende festlegt.

11. Uterusmanipulator nach einem der vorhergehenden Ansprüche, **dadurch gekennzeichnet dass** die Hohlsonde (1) das Griffteil (4) proximal überragt und dort mit einer Skalierung (9) versehen ist, welche die freie Länge des distalen Endabschnitts (2) entsprechend der axialen Position der Hohlsonde (1) anzeigt.

12. Uterusmanipulator nach einem der vorhergehenden Ansprüche, **dadurch gekennzeichnet dass** der Saugkanal (10) als ein zentraler Saugkanal ausgebildet ist und der Saugkanal (12) in der Glocke (12) als ein Ringkanal ausgebildet ist, wobei die Hohlsonde (1) den zentralen Saugkanal (10) aufweist, welcher im Endbereich des Schaftes (3) über eine Querdurchbrechung in der Sondenwandung in einen zwischen der Hohlsonde (1) und dem Schaft (3) gebildeten Ringkanal (12) mündet, der durch einen O-Ring abgedichtet ist und proximalseitig an eine Saugleitung anschließt.

13. Uterusmanipulator nach einem der vorhergehenden Ansprüche, **dadurch gekennzeichnet dass** im oder am Schaft (3) mindestens ein Lichtleiter (21) angeordnet ist, der an der Stirnfläche (18) nahe dem Innenumfang der Saugglocke (13) mündet.

14. Uterusmanipulator nach einem der vorhergehenden Ansprüche, **dadurch gekennzeichnet dass** in oder an der Saugglocke (13) Leuchtdioden (20) angeordnet sind und dass im oder am Schaft (3) mindestens eine elektrische Versorgungsleitung (26) geführt ist.

## Claims

1. A uterus manipulator, in particular for laparoscopically assisted vaginal hysterectomy, with a shank (3), with a distal end section (2) which is envisaged and designed for introduction into a uterus through the cervical canal, with a proximal handle (4), with a distally open bell (13) for receiving a cervix and with a vaginal seal (29) which with regard to the bell (13) is arranged proximally displaced thereto, with at least one suction channel (10) and with at least one suction opening (11) in the distal end section (2), **characterised in that** the bell (13) is designed as a suction bell (13) and that at least one suction channel (12) runs out in the bell (13), wherein the vaginal seal (29) is formed by a proximally open sealing bell (28) whose peripheral edge (29) is designed for the sealing bearing contact on the vaginal wall and wherein the suction bell (13) and the sealing bell (28) form a construction unit or part of a construction unit, which is releasably fastened on the shank (3).

2. A uterus manipulator according to claim 1, **characterised in that** the suction bell (13) comprises at least one step (14) on its inner periphery.

3. A uterus manipulator according to one of the preceding claims, **characterised in that** illumination means (20; 21) for illuminating the peripheral, distal edge (19) of the suction bell (13) are provided.

4. A uterus manipulator according to one of the preceding claims, **characterised in that** the suction bell (13) consists of material leading light, is designed truncated-cone-shaped on the outside and multi-stepped on the inside, wherein the light impingement is effected at an end-face (18) close to the shank-side inner periphery.

5. A uterus manipulator according to one of the preceding claims, **characterised in that** the construction unit comprises an intrinsically stiff component which forms at least the suction bell (13), and a soft-elastic component forming at least the vaginal seal (29).

6. A uterus manipulator according to one of the preceding claims, **characterised in that** the component comprising the suction bell (13) is designed as a plastic injection moulded part.

7. A uterus manipulator according to one of the preceding claims, **characterised in that** a hollow probe (1) is guided in the shank (3) in a rotationally fixed and axially displaceable manner and comprises the distal end section (2) for introduction into the cervical canal and can be fixed in at least two different axial positions with respect to the shank (3) and/or the proximal handle (4).

8. A uterus manipulator according to one of the preceding claims, **characterised in that** the suction bell (13), in particular the construction unit, is fastened close to the distal shank end on this.

9. A uterus manipulator according to one of the preceding claims, **characterised in that** the shank (3) on the proximal side carries a grip part (4) which forms the handle of the instrument.

10. A uterus manipulator according to one of the preceding claims, **characterised in that** the hollow probe (1) comprises locking recesses (7) on the outer periphery and that a spring-biased locking body (6) arranged in the grip part (4) engages into one of the recess (7) and thus fixes the axial extension of the hollow probe (1) from the grip part (4) and/or from the distal shank end.

11. A uterus manipulator according to one of the preceding claims, **characterised in that** the hollow probe (1) projects proximally beyond the grip part (4) and is provided with a scale (9) there, said scale indicating the free length of the distal end section (2) according to the axial position of the hollow probe (1).

12. A uterus manipulator according to one of the preceding claims, **characterised in that** the suction channel (10) is designed as a central suction channel, and the suction channel (12) in the bell (13) is designed as an annular channel, wherein the hollow probe (1) comprises the central suction channel (10) which in the end region of the shank (3), via a transverse opening in the probe wall, runs out into the annular channel (12) which is formed between the hollow probe (1) and the shank (3), is sealed by an O-ring and connects at the proximal side to a suction conduit.

13. A uterus manipulator according to one of the preceding claims, **characterised in that** at least one fibre-optic (21) is arranged in or on the shank (3), said fibre-optic running out at the end-face (18) close to the inner periphery of the suction bell (13).

14. A uterus manipulator according to one of the preceding claims, **characterised in that** light diodes (20) are arranged in or on the suction bell (13) and that at least one electrical supply lead (26) is led in or on the shank (3).

## Revendications

1. Manipulateur utérin, en particulier pour l'hystérectomie vaginale assistée par laparoscopie, comprenant une tige (3), une partie d'extrémité distale (2) qui est destinée et conçue pour être introduite dans un utérus à travers le canal cervical, une poignée (4) proximale, une cloche (13) ouverte en direction distale pour recevoir le col de l'utérus, et un anneau d'étanchéité vaginal (29) disposé de façon décalée par rapport à la cloche (13) en direction proximale, au moins un canal d'aspiration (10) et au moins une ouverture d'aspiration (11) dans la partie d'extrémité distale (2), **caractérisé en ce que** la cloche (13) est réalisée en tant que cloche d'aspiration et **en ce qu'**au moins un canal d'aspiration (12) débouche dans la cloche (13), l'anneau d'étanchéité vaginal (29) étant formé par une cloche d'étanchéité (28) ouverte en direction proximale, dont le bord (29) périphérique est conçu pour être en appui étanche contre la paroi vaginale, et la cloche d'aspiration (13) et la cloche d'étanchéité (28) formant une unité ou une partie d'une unité qui est fixée de façon amovible sur la tige (3).

2. Manipulateur utérin selon la revendication 1, **caractérisé en ce que** la cloche d'aspiration (13) présente au moins un gradin (14) sur sa périphérie intérieure.

3. Manipulateur utérin selon l'une des revendications précédentes, **caractérisé en ce que** sont prévus des moyens d'éclairage (20; 21) pour éclairer le bord périphérique distal (19) de la cloche d'aspiration (13).

4. Manipulateur utérin selon l'une des revendications précédentes, **caractérisé en ce que** la cloche d'aspiration (13) est constituée d'un matériau conduisant la lumière, présente une forme tronconique à l'extérieur et une configuration à gradins multiples à l'intérieur, l'éclairement s'opérant au niveau d'une surface avant (18) à proximité de la périphérie intérieure côté tige.

5. Manipulateur utérin selon l'une des revendications précédentes, **caractérisé en ce que** l'unité présente un composant à rigidité intrinsèque formant au moins la cloche d'aspiration (13) et un composant élastique souple formant au moins l'anneau d'étanchéité vaginal (29).

6. Manipulateur utérin selon l'une des revendications précédentes, **caractérisé en ce que** le composant comprenant la cloche d'aspiration (13) est réalisé sous forme de pièce moulée par injection de matière plastique.

7. Manipulateur utérin selon l'une des revendications précédentes, **caractérisé en ce qu'**est guidée dans la tige (3), de manière solidaire en rotation et déplaçable axialement, une sonde creuse (1) qui présente la partie d'extrémité distale (2) pour introduction dans le canal cervical et peut être fixée dans au moins deux positions axiales différentes par rapport à la tige (3) et/ou à la poignée (4) proximale.

8. Manipulateur utérin selon l'une des revendications précédentes, **caractérisé en ce que** la cloche d'aspiration (13), en particulier l'unité, est fixée à proximité de l'extrémité distale de la tige sur celle-ci.

9. Manipulateur utérin selon l'une des revendications précédentes, **caractérisé en ce que** la tige (3) porte côté proximal une partie poignée (4) qui forme la poignée de l'instrument.

10. Manipulateur utérin selon l'une des revendications précédentes, **caractérisé en ce que** la sonde creuse (1) présente, sur la périphérie extérieure, des évidements d'encliquetage (7) et **en ce qu'**un corps d'encliquetage (6) précontraint par ressort et disposé dans la partie poignée (4) vient en prise dans l'un des évidements (7) et définit ainsi l'étendue axiale de la sonde creuse (1) à partir de la poignée (4) et/ou de l'extrémité de tige distale.

11. Manipulateur utérin selon l'une des revendications précédentes, **caractérisé en ce que** la sonde creuse (1) dépasse la partie poignée (4) côté proximal et est pourvue à cet endroit d'une graduation (9) qui indique la longueur libre de la partie d'extrémité distale (2) en fonction de la position axiale de la sonde creuse (1).

12. Manipulateur utérin selon l'une des revendications précédentes, **caractérisé en ce que** le canal d'aspiration (10) est réalisé en tant que canal d'aspiration central et **en ce que** le canal d'aspiration (12) dans la cloche (13) est réalisé sous la forme d'un canal annulaire, la sonde creuse (1) comportant le canal d'aspiration (10) central qui, dans la région d'extrémité de la tige (3), par l'intermédiaire d'une découpe transversale ménagée dans la paroi de la sonde, débouche dans un canal annulaire (12) formé entre la sonde creuse (1) et la tige (3), canal qui est rendu étanche par un joint torique et se raccorde côté proximal à un conduit d'aspiration.

13. Manipulateur utérin selon l'une des revendications précédentes, **caractérisé en ce qu'**est disposé dans ou sur la tige (3) au moins un guide de lumière (21) qui débouche sur la face avant (18) à proximité de la périphérie intérieure de la cloche d'aspiration (13).

14. Manipulateur utérin selon l'une des revendications précédentes, **caractérisé en ce que** des diodes lumineuses (20) sont disposées dans ou sur la cloche d'aspiration (13) et **en ce qu'**au moins un conduit d'alimentation électrique (26) est guidé dans ou sur la tige (3).
